# FASCICULE DE BREVET EUROPEEN

(11) **EP 1 934 333 B1**
(45) Date de publication et mention de la délivrance du brevet: **02.11.2011**
(21) Numéro de dépôt: 06808166.0
(22) Date de dépôt: 19.09.2006
(51) Int. Cl.: C12N 5/077

(54) **PROCEDE D'OBTENTION DE CELLULES MUSCULAIRES LISSES HUMAINES ET LEURS APPLICATIONS**
VERFAHREN ZUR GEWINNUNG MENSCHLICHER GLATTER MUSKELZELLEN UND VERWENDUNGEN DAVON
METHOD FOR OBTAINING HUMAN SMOOTH MUSCULAR CELLS AND USES THEREOF

(30) Priorité: 19.09.2005 FR 0509557
(43) Date de publication de la demande: 25.06.2008
(73) Titulaire: Assistance Publique - Hôpitaux de Paris, 75004 Paris (FR); INSTITUT DES VAISSEAUX ET DU SANG, 75475 Paris Cedex 10 (FR)
(72) Inventeur: LE RICOUSSE, Sophie, F-94500 Champigny (FR); LACASSAGNE, Marie-Noëlle, F-75018 Paris (FR); MAROLLEAU, Jean-Pierre, F-80000 Amiens (FR)
(74) Mandataire: Warcoin, Jacques
(86) Numéro de dépôt international: PCT/FR2006/002144
(87) Numéro de publication internationale: WO 2007/034069

(56) Documents cités:
- WO-A-01/94555
- LE RICOUSSE-ROUSSANNE SOPHIE ET AL: "Ex vivo differentiated endothelial and smooth muscle cells from human cord blood progenitors home to the angiogenic tumor vasculature." CARDIOVASCULAR RESEARCH, vol. 62, no. 1, 1 avril 2004 (2004-04-01), pages 176-184, XP002387433 ISSN: 0008-6363 cité dans la demande
- SIMPER DAVID ET AL: "Smooth muscle progenitor cells in human blood." CIRCULATION, vol. 106, no. 10, 3 septembre 2002 (2002-09-03), pages 1199-1204, XP002387434 ISSN: 1524-4539
- HWANG JI HYE ET AL: "Isolation of muscle derived stem cells from rat and its smooth muscle differentiation [corrected]." MOLECULES AND CELLS, vol. 17, no. 1, 29 février 2004 (2004-02-29), pages 57-61, XP002387435 ISSN: 1016-8478 cité dans la demande
- QU-PETERSEN Z ET AL: "Identification of a novel population of muscle stem cells in mice: Potential for muscle regeneration" JOURNAL OF CELL BIOLOGY, vol. 157, no. 5, 27 mai 2002 (2002-05-27), pages 851-864, XP002302343 ISSN: 0021-9525
- HUARD J ET AL: "Muscle-derived cell-mediated ex vivo gene therapy for urological dysfunction" GENE THERAPY, MACMILLAN PRESS LTD., BASINGSTOKE, GB, vol. 9, no. 23, décembre 2002 (2002-12), pages 1617-1626, XP002370482 ISSN: 0969-7128

### LISTE DE SEQUENCES

<110> ASSISTANCE PUBLIQUE - HOPITAUX DE PARIS
   INSTITUT DES VAISSEAUX ET DU SANG
<120> PROCEDE D'OBTENTION DE CELLULES MUSCULAIRES LISSES HUMAINES ET LEURS
   APPLICATIONS
<130> D23375
<150> FR 05/09557
   <151> 2005-09-19
<160> 18
<170> PatentIn version 3.3
<210> 1
   <211> 18
   <212> ADN
   <213> séquence artificielle
<220>
   <223> séquence amorce sens gène GAPDH
<400> 1
   ccatggagaa ggctgggg 18
<210> 2
   <211>
   <212> ADN
   <213> séquence artificielle
<220>
   <223> séquence amorce antisens gène GAPDH
<400> 2
   caaagttgtc atggatgacc 20
<210> 3
   <211> 18
   <212> ADN
   <213> séquence artificielle
<220>
   <223> séquence amorce sens gène calponine
<400> 3
   agaagtatga ccaccagc 18
<210> 4
   <211> 20
   <212> ADN
   <213> séquence artificielle
<220>
   <223> séquence amorce antisens gène calponine
<400> 4
   tagagcccaa tgatgttccg 20
<210> 5
   <211> 20
   <212> ADN
   <213> séquence artificielle
<220>
   <223> séquence amorce sens gène SM22α
<400> 5
   gcagtccaaa attgagaaga 20
<210> 6
   <211> 20
   <212> ADN
   <213> séquence artificielle
<220>
   <223> séquence amorce antisens gène SM22α
<400> 6
   ctgttgctgc ccatttgaag 20
<210> 7
   <211> 18
   <212> ADN
   <213> séquence artificielle
<220>
   <223> séquence amorce sens gène Myogénine
<400> 7
   agcgccccct cgtgtatg 18
<210> 8
   <211> 19
   <212> ADN
   <213> séquence artificielle
<220>
   <223> séquence amorce antisens gène Myogénine
<400> 8
   tgtccccggc aacttcagc 19
<210> 9
   <211> 21
   <212> ADN
   <213> séquence artificielle
<220>
   <223> séquence amorce sens gène MyoD
<400> 9
   cgccggcgga actgctacga a 21
<210> 10
   <211> 19
   <212> ADN
   <213> séquence artificielle
<220>
   <223> séquence amorce antisens gène MyoD
<400> 10
   ggggcggggg cggaaactt 19
<210> 11
   <211> 19
   <212> ADN
   <213> séquence artificielle
<220>
   <223> séquence amorce sens gène Myf5
<400> 11
   accatggatc ggcggaagg 19
<210> 12
   <211> 21
   <212> ADN
   <213> séquence artificielle
<220>
   <223> séquence amorce antisens gène Myf5
<400> 12
   aatcggtgct gccaactgga g 21
<210> 13
   <211> 21
   <212> ADN
   <213> séquence artificielle
<220>
   <223> séquence amorce sens gène VEGF-R1
<400> 13
   cgaccttggt tgtggctgac t 21
<210> 14
   <211> 21
   <212> ADN
   <213> séquence artificielle
<220>
   <223> séquence amorce antisens gène VEGF-R1
<400> 14
   cccttctggt tggtggcttt g 21
<210> 15
   <211> 18
   <212> ADN
   <213> séquence artificielle
<220>
   <223> séquence amorce sens gène VEGF-R2
<400> 15
   aacaaagtcg ggagagga 18
<210> 16
   <211> 21
   <212> ADN
   <213> séquence artificielle
<220>
   <223> séquence amorce antisens gène VEGF-R2
<400> 16
   agacaagaag tagccagaag a 21
<210> 17
   <211> 20
   <212> ADN
   <213> séquence artificielle
<220>
   <223> séquence amorce sens gène SRF
<400> 17
   agtgtgtggg ggagattctg 20
<210> 18
   <211> 20
   <212> ADN
   <213> séquence artificielle
<220>
   <223> séquence amorce antisens gène SRF
<400>, 18
   tctccctagc aacagcccta 20

## Description

La présente invention est relative à un procédé d'obtention *in vitro* d'une population de cellules comprenant essentiellement des cellules musculaires lisses humaines (hCML) exprimant la calponine et la SM-MHC à partir d'un échantillon de cellules de biopsie musculaire humaine ou à partir de cellules de biopsies musculaires humaines différenciées *in vitro* en cellules musculaires squelettiques (hCMS).

Les cellules musculaires lisses (CML), présentes dans les vaisseaux, les intestins et la vessie, et les cellules musculaires squelettiques (CMS) sont les deux types cellulaires utilisés par l'organisme pour remplir la fonction de contraction mécanique. L'origine des CML est complexe et dépend de leur localisation. En effet au cours de l'embryogenèse, les précurseurs des CML peuvent provenir de trois lignages : les cellules mésenchymateuses, les cellules de la crête neurale ou les cellules dérivant de l'épicarde. Récemment, l'existence de progéniteurs de CML circulant dans le sang périphérique à été observée. En effet, différents modèles animaux utilisés pour étudier (i) la formation néointimale des vaisseaux, (ii) le devenir des greffes artérielles ou la formation des plaques d'athérosclérose, ont permis de mettre en évidence la participation des progéniteurs contenus dans les cellules de la moelle à ces processus et leur différenciation en CML.

Chez l'adulte, la réparation des fibres musculaires squelettiques est assurée par la population des cellules satellites, cellules myogéniques mononucléées situées sous la lame basale des fibres musculaires. Mais il semble que cette population cellulaire soit hétérogène. De plus d'autres cellules, multipotentes, isolées du muscle squelettique par cytométrie en flux selon leur propriété à libérer le colorant Hoechst (1, 2), sont capables de se différencier en toutes les cellules du sang quand elles sont transplantées chez des souris dont la moelle osseuse a été détruite par irradiation (2). Cette population cellulaire a été appelée « side population » (SP). Elle est définie par l'expression du marqueur Scal. En revanche elle n'exprime pas le CD34, le ckit et le CD45. Ces cellules sont capables de se différencier en cellules de muscle desmine+ dans des conditions de cultures appropriées (1). D'autres travaux décrivent l'existence de cellules précurseurs dans le muscle squelettique ayant des propriétés de « plasticité » cellulaire importantes (3). Le muscle squelettique semble donc contenir plusieurs types de cellules souches ayant des propriétés de multipotences variées.

La définition des propriétés de différenciation de ces cellules souches et leur contrôle en culture permettraient d'utiliser ces cellules, facilement isolables, en thérapie notamment en thérapie réparatrice. Ces cellules, cultivées ex vivo, pourront alors être transplantées constituant un produit de thérapie cellulaire pour le traitement en autologue des pathologies vasculaires (revascularisation post-ischémique, athérosclérose, stabilisation des vaisseaux tumoraux, ...).

La différenciation des CMS a déjà été décrite chez le rat par Hwang JH. et a1. (4) en utilisant un procédé mettant en oeuvre une coculture de CMS avec des CML de vessie et en présence de VEGF, ce procédé permettant d'obtenir des CMS différenciées exprimant l'aSMA.

On peut également citer la demande de brevet internationale publiée sous le No. WO 03/027281 (Sakurada Kazuhiro et a1.) décrivant l'obtention d'une population de cellules souches multipotentes provenant du tissu intersticiel du muscle squelettique capable de se différencier en neurones, cellules gliales, cellules du muscle cardiaque, adipocytes, cellules de l'endothélium vasculaire, cellules du sang, cellules osseuses, cellules du cartilage, cellules du pancréas et cellules hépatiques.

On peut enfin citer la demande de brevet internationale publiée sous le No. WO 01/94555 (J.P. Marolleau et a1.) décrivant un procédé d'obtention de populations cellulaires caractérisées d'origine musculaire et leurs utilisations. Ce document décrit en particulier un procédé d'obtention d'une population de cellules dont le type cellulaire dominant exprime le marqueur CD56 et le marqueur HLA de classe I, à partir d'une biopsie de tissu musculaire, pour la préparation d'un produit de thérapie cellulaire apte à l'administration humaine, notamment de transplant pour potentialiser les traitements pharmacologiques des insuffisances cardiaques.

Il serait donc souhaitable de pouvoir disposer d'un procédé d'obtention *in vitro* d'une population de cellules comprenant essentiellement des cellules musculaires lisses (CML), notamment à partir d'un échantillon de tissu musculaire issu d'individu ou de patient que l'on souhaite traiter à l'aide de cette population.

Ceci est justement l'objet de la présente invention.

Ainsi, sous un premier aspect, la présente invention a pour objet un procédé d'obtention *in vitro* d'une population de cellules comprenant essentiellement des cellules musculaires lisses humaines (hCML) exprimant la calponine et la chaîne lourde de la myosine musculaire lisse, dénommée ici SM-MHC (« SM-MHC » pour « Smooth Muscle Myosin Heavy Chains »), à partir d'un échantillon de cellules de biopsie usculaire squelettique humaine ou à partir de cellules de biopsies musculaires humaines différenciées *in vitro* en cellules musculaires squelettiques (hCMS),
- lesdites cellules de biopsies musculaires humaines n'exprimant pas le CD31 et le CD14, et, le cas échéant, les marqueurs des lymphocytes B et T, et
- lesdites hCMS exprimant le CD56, la desmine et un gène de la myogénèse choisi dans le groupe de gènes constitué du gène MyoD, Myf5 et la myogénine, et sont capables de générer des myotubes multinucléés,
   caractérisé en ce qu'il comprend les étapes suivantes :
   A) la mise en culture desdites cellules de biopsies musculaires humaines myoblastiques dans un milieu de culture comprenant du VEGF (facteur de croissance de l'endothélium vasculaire), de préférence du VEGF de séquence humaine, ladite culture étant de préférence réalisée en absence de CML issues de vessie ; et
   B) la récupération des hCML obtenues à l'étape A).

Par le terme « essentiellement » dans l'expression « comprenant essentiellement des cellules musculaires lisses humaines (hCML) », on entend désigner ici notamment une population contenant au moins 50 %, de préférence à au moins 60 %, 70 %, 75 % et 80 % de hCML par rapport à la population cellulaire globale obtenue.

De préférence, le procédé selon l'invention est caractérisé en ce que lesdites hCMS exprimant le CD56 et la desmine, expriment les gènes MyoD, Myf5 et la myogénine.

De préférence, le procédé selon l'invention est caractérisé en ce que lesdites hCMS n'expriment pas le CD34 et le CD 14.

De préférence, le procédé selon l'invention est caractérisé en ce que lesdites hCMS n'expriment pas la calponine et la SM-MHC.

De préférence, le procédé selon l'invention est caractérisé en ce que lesdites hCML obtenues à l'étape A) expriment la calponine et la SM-MHC.

De préférence, le procédé selon l'invention est caractérisé en ce que lesdites hCML obtenues à l'étape A) n'expriment pas le gène MyoD.

De préférence, le procédé selon l'invention est caractérisé en ce que lesdites hCML obtenues à l'étape A) expriment Myf5 et la myogénine.

De préférence, le procédé selon l'invention est caractérisé en ce que ledit milieu de culture utilisé à l'étape A) comprend en outre au moins un facteur de croissance, de préférence de séquence humaine, choisi dans le groupe de facteurs de croissance constitué du PDGF-BB (facteur de croissance dérivé des plaquette, homodimère BB, dénommé encore homodimère bb), IGF1 (facteur de croissance apparenté à l'insuline de type 1), FGFb (facteur de croissance fibroblastique basique), HGF (facteur de croissance des hépatocytes) et TNFa (facteur nécrosant les tumeurs de type alpha), GFβ.

De préférence, le procédé selon l'invention est caractérisé en ce que lesdites hCML sont obtenues à l'étape A) à partir d'un échantillon de cellules de biopsies musculaires humaines différenciées *in vitro* en cellules musculaires squelettiques (hCMS), caractérisé en ce que lesdites hCMS sont obtenues à partir d'un échantillon de cellules de biopsie musculaire humaine par un procédé comprenant les étapes suivantes :
a) éminçage de ladite biopsie musculaire,
b) dissociation enzymatique des fibres et cellules musculaires et séparation des cellules individualisées par filtration,
c) mise en culture des cellules d'origine musculaire ainsi obtenues dans un réacteur de culture de cellules adhérentes en présence d'un milieu de croissance et/ou de différenciation suivie le cas échéant d'une ou plusieurs phases d'expansion,
d) identification des types cellulaires présents aux différents stades de la culture par l'analyse de marqueurs cellulaires spécifiques,
e) choix du stade de culture pendant lequel le type cellulaire recherché est en proportion dominante dans la population de cellules,
f) récolte d'une population de cellules au stade de culture choisi en e),
g) le cas échéant, congélation des cellules prélevées à l'étape f), notamment au stade de culture qui sera choisi pour la préparation du produit de thérapie cellulaire.

Selon un mode préféré du procédé ci-avant selon l'invention, on effectue :
- à l'étape b) :
   - un lavage des éminçats dans un milieu A suivi de la dissociation enzymatique en présence de libérase dudit éminçat ;
   - la séparation des cellules individualisées ainsi obtenues par filtration sur tamis suivie d'une centrifugation ; et
   - le lavage dans un milieu B du culot cellulaire ainsi obtenu,
- à l'étape c) :
   - la mise en culture des cellules obtenues à l'étape b) sur un plateau de culture dans un milieu C jusqu'à obtenir un degré de confluence d'environ 20 à 50 % ou jusqu'à l'apparition des premiers myotubes, puis le lavage des cellules en tampon PBS (tampon phosphate salin), en SVF (sérum de veau foetal) puis en milieu C, la culture en milieu C sur des unités de plateaux élargis ou multi-étagés pouvant être de nouveau réalisée pour atteindre un degré de confluence d'environ 90 % ou l'apparition des premiers myotubes ;
   - l'élimination du milieu de culture C et son remplacement par un milieu D la veille de la récolte desdites cellules ; et
   - le lavage des cellules ainsi obtenues en PBS puis en milieu A,
- le cas échéant, à la fin de l'étape f) :
   - la concentration desdites cellules ainsi obtenues en milieu A supplémentée de sérum albumine humaine à 0,5 % (P/V), et
- à l'étape g) :
   - la congélation desdites cellules ainsi obtenues à l'étape f) est réalisée en milieu A supplémenté de sérum albumine humaine à 4 % (P/V) et en DMSO à 7,5 % (V/V ), leur décongélation à 37°C, puis après un lavage en milieu A, leur suspension dans le milieu de culture,
      et dans lesquelles étapes lesdits milieux A, B, C et D sont les milieux tels que définis dans la demande internationale de brevet publiée sous le No. WO 01/94555 le 13 décembre 2001 (pages 24 et 25) à savoir :
      Milieu A :
         - Milieu MCDB 120 (Ham et al., 1988) modifié : substitution de la L-valine par de la D-valine, élimination du rouge de phénol et de la thymidine.
      Milieu B :
         - Milieu A + 20 % de sérum de veau foetal irradié + antibiotique.
      Milieu C :
         - Milieu B + FGFb (10 ng/ml) + dexaméthasone à 1 µM. Solution D : Tampon phosphate salin (PBS).

De préférence, l'antibiotique utilisé est de la gentamycine, notamment à 50 µg par ml, ou un mélange de pénicilline et de streptomycine (notamment à 100 UI/ml et 100 µg/ml respectivement).

Dans un mode de réalisation encore préféré, le procédé selon l'invention est caractérisé en ce que lesdites hCML sont obtenues à partir d'un échantillon de cellules de biopsie musculaire humaine préalablement prédifférenciées en hCMS obtenues selon le procédé tel que décrit dans la demande internationale de brevet publiée sous le No. WO 01/94555, et dans lequel procédé, le stade de culture pendant lequel le type cellulaire hCMS recherché est en proportion significative dans la population de cellules, est déterminé par l'apparition d'une population cellulaire de phénotype CD56+ représentant au moins 50 %, de préférence à au moins 60 %, 70 %, 75 % et 80 % de la population générale.

De préférence, ladite population cellulaire de phénotype CD56+ représentant au moins 50 %, de préférence à au moins 60 %, 70 %, 75 % et 80 % de la population générale possède en outre l'un au moins des phénotypes, de manière préférée au moins 2, 3 et les 4 phénotypes, choisi dans le groupe de phénotypes constitué de CD10+, CD13+, desmine+, HLA de classe 1 et n'exprimant pas le HLA de classe 2.

Dans un mode de réalisation préféré, le procédé d'obtention *in vitro* d'une population de cellules comprenant essentiellement des hCML selon l'invention et dans lequel procédé lesdites hCML sont obtenues à partir d'un échantillon de cellules de biopsies musculaires humaines différenciées *in vitro* en cellules musculaires squelettiques (hCMS), est caractérisé en ce que à l'étape A), ledit milieu de culture comprenant du VEGF est le milieu MCDB 120 tel que décrit par Ham et al. (in vitro Cell Dev. Biol., 24, 833-844, 1998) et modifié par substitution de la L-Valine par la D-Valine, élimination du rouge de phénol et de la thymidine.

Dans un mode de réalisation préféré, le procédé d'obtention *in vitro* d'une population de cellules comprenant essentiellement des hCML selon l'invention et dans lequel procédé lesdites hCML sont obtenues à partir d'un échantillon de cellules de biopsies musculaires humaines, est caractérisé en ce qu'à l'étape A), ledit milieu de culture comprenant du VEGF est le milieu M199 (comme par exemple Medium 199 Gibco, Grand Island, NY).

Dans un mode de réalisation également préféré, le procédé d'obtention *in vitro* d'une population de cellules comprenant essentiellement des hCML selon l'invention est caractérisé en ce qu'à l'étape A), ledit milieu de culture comprend 10 ng/ml de VEGF.

Dans un mode de réalisation également préféré, le procédé d'obtention *in vitro* d'une population de cellules comprenant essentiellement des hCML selon l'invention est caractérisé en ce que la biopsie musculaire humaine à partir de laquelle lesdites hCML sont obtenues directement ou préalablement prédifférenciées en hCMS, est une biopsie prélevée dans tout territoire musculaire, de préférence dans le territoire musculaire de la jambe, de l'individu, individu enfant ou adulte, chez qui le prélèvement est effectué.

Sous un autre aspect, la présente demande décrit les cellules musculaires lisses humaines isolées susceptibles d'être obtenues par le procédé selon l'invention, lesdites cellules musculaires lisses humaines isolées étant caractérisées en ce qu'elles expriment la calponine et la SM-MHC.

Sous encore un autre aspect, la présente demande déc une composition comprenant des cellules musculaires lisses humaines isolées susceptibles d'être obtenues ou directement obtenues à partir d'un échantillon de cellules de biopsie musculaire humaine ou à partir de cellules de biopsies musculaires humaines différenciées *in vitro* en cellules musculaires squelettiques par le procédé selon l'invention, à titre de médicament.

La présente demande décrit également l'utilisation de cellules musculaires lisses humaines isolées susceptibles d'être obtenues ou directement obtenues à partir d'un échantillon de cellules de biopsie musculaire humaine ou à partir de cellules de biopsies musculaires humaines différenciées *in vitro* en cellules musculaires squelettiques par le procédé selon l'invention, ou l'utilisation de la composition à titre de médicament selon l'invention pour la préparation d'une composition thérapeutique destinée à l'Homme, notamment destinée à l'individu dont sont originaires les cellules de biopsie musculaire cultivées à l'étape A) dudit procédé.

Ladite composition thérapeutique peut être destinée au remplacement ou à la transplantation de CML chez l'Homme, de préférence le remplacement ou la transplantation est de type autologue.

Ladite composition thérapeutique peut être destinée à la prévention ou au traitement de cancers, de préférence en administration préalable ou simultanée à un traitement anti-cancéreux par chimiothérapie ou par radiothérapie

En effet, contrairement aux vaisseaux normaux, les vaisseaux tumoraux sont structuralement et fonctionnellement différents. L'identification des marqueurs spécifiques des vaisseaux tumoraux permettrait de cibler ces vaisseaux sans détruire la vasculature normale (thérapie anti-angiogénique) (5). De nombreuses études ont démontré les altérations fonctionnelles des cellules endothéliales (CE) des vaisseaux tumoraux. Et des résultats récents démontrent que les cellules péri-vasculaires (péricytes ou CML) subissent des modifications phénotypiques et fonctionnelles (forme anormale, expression de nouveaux marqueurs, association faible avec les CE, possèdent une extension cytoplasmique qui pénètre profondément dans le parenchyme tumoral) dans le microenvironnement tumoral (6-8), devenant ainsi une nouvelle cible pour les thérapies anti-angiogéniques. Ces caractéristiques physiopathologiques des tumeurs solides compromettent la délivrance et l'efficacité des thérapies cytotoxiques classiques et des thérapies ciblées. Une nouvelle approche thérapeutique serait de rendre normale la vasculature tumorale avant sa destruction pour faciliter la délivrance de drogues (pour revue voir (9)). De fait, des résultats récents démontrent l'efficacité de la régression de la tumeur, à l'aide de thérapies combinées, après stabilisation et normalisation de la vasculature tumorale (10). Cette stabilisation des vaisseaux tumoraux pourrait être réalisée en injectant les CML au site de la tumeur ou en périphérie.

Cette approche thérapeutique (l'injection des CML humaines isolées susceptibles d'être obtenues ou directement obtenues par le procédé selon l'invention, en vue d'une normalisation des vaisseaux tumoraux) ne sera effectuée de préférence qu'en association avec une chimiothérapie ou une radiothérapie. Il faudra pour cela définir une « fenêtre thérapeutique », période au cours de laquelle l'injection des CML permettra l'effet des traitements anti-cancéreux le plus grand.

La « normalisation » vasculaire assurera un réseau plus fonctionnel, favorisant ainsi la diffusion locale des drogues, leur distribution plus homogène et l'oxygénation de la tumeur nécessaire au fonctionnement de certaines drogues. Cela permettra une action plus rapide et plus étendue des drogues dans la tumeur, donc une diminution des doses administrées réduisant à priori la sévérité et la fréquence des effets secondaires. Finalement, la rapidité et la combinaison des actions limiteront rapidement la prolifération et donc les phénomènes de résistance tumorale souvent observés.

La thérapie cellulaire proposée ici, ne constitue pas une nouvelle forme de traitement destinée à remplacer les traitements actuels, mais interviendra comme -complément-et/ou synergie potentielle aux chimiothérapies et radiothérapies proposées actuellement.

Ladite composition thérapeutique peut être destinée à la prévention ou au traitement de l'ischémie, en particulier cardiaque ou des membres inférieurs.

De nombreuses études réalisées chez la souris et quelques protocoles chez l'humain, ont permis de mettre en évidence l'amélioration de la revascularisation post-ischémique (ischémie cardiaque ou des membres inférieurs) après l'injection de cellules de la moelle ou des cellules différenciées *in vitro.* Si actuellement une réelle intégration de ces. cellules aux néovaisseaux semble être remise en cause, les effets fonctionnels observés sont réels. De plus, le rôle des CML dans ces processus pourrait être très important. Des résultats récents démontrent, au site de néovascularisation, la différenciation des cellules de la moelle injectées à des souris, uniquement en cellules périendothéliales, et non pas en cellules endothéliales (11).

Ainsi, de manière plus particulière, la présente demande décrite l'utilisation de cellules musculaires lisses humaines isolées susceptibles d'être obtenues ou directement obtenues à partir d'un échantillon de cellules de biopsie musculaire humaine ou à partir de cellules de biopsies musculaires humaines différenciées *in vitro* en cellules musculaires squelettiques par le procédé selon l'invention, pour la préparation d'une composition destinée à la « normalisation » de la vasculature tumorale ou à la revascularisation post-ischémique.

Cependant ces cellules pourraient être utilisées aussi comme médicament destiné à un usage en thérapeutique pour : l'athérosclérose, les maladies veineuses chroniques, les malformations vasculaires (tels que les angiomes).

C'est pourquoi, la présente demande décrit de cellules musculaires lisses humaines isolées susceptibles d'être obtenues ou directement obtenues à partir d'un échantillon de cellules de biopsie musculaire humaine ou à partir de cellules de biopsies musculaires humaines différenciées *in vitro* en cellules musculaires squelettiques par le procédé selon l'invention, pour la préparation d'une composition destinée à la prévention ou au traitement de l'athérosclérose, des arthérites, des maladies veineuses chroniques ou des malformations vasculaires, en particulier des angiomes.

Enfin, de par leurs propriétés de migration vers un site de néoangiogenèse, ces cellules pourront être utilisées comme navette ou vecteur afin de délivrer des principes actifs thérapeutiques comme des drogues ou des facteurs anti ou pro-angiogéniques.

Ainsi, sous un autre aspect particulier, la présente demande décrite l'utilisation de cellules musculaires lisses humaines isolées susceptibles d'être obtenues ou directement obtenues à partir d'un échantillon de cellules de biopsie musculaire humaine ou à partir de cellules de biopsies musculaires humaines différenciées *in vitro* en cellules musculaires squelettiques par le procédé selon l'invention, à titre de médicament, notamment à titre de vecteur pour l'administration de principe actif ou de composé thérapeutique, caractérisée en ce que:
- lesdites cellules musculaires lisses humaines isolées sont transformées de manière à pouvoir exprimer ledit principe actif ou composé thérapeutique ; ou
- lesdites cellules musculaires lisses humaines isolées ont été modifiées afin de contenir ledit principe actif ou composé thérapeutique que l'on souhaite administrer.

Est également décrit dans la présente demande l'utilisation de cellules musculaires lisses humaines isolées susceptibles d'être obtenues ou directement obtenues à partir d'un échantillon de cellules de biopsie musculaire humaine ou à partir de cellules de biopsies musculaires humaines différenciées *in vitro* en cellules musculaires squelettiques par le procédé selon l'invention, lesdites cellules étant capables d'exprimer un principe actif ou composé thérapeutique ou contenant un principe actif ou composé thérapeutique, pour la préparation d'une composition thérapeutique destinée à la prévention ou au traitement de maladies nécessitant un traitement par ledit principe actif ou composé thérapeutique.

L'utilisation de cellules musculaires lisses humaines isolées susceptibles d'être obtenues ou directement obtenues à partir d'un échantillon de cellules de biopsie musculaire humaine ou à partir de cellules de biopsies musculaires humaines différenciées *in vitro* en cellules musculaires squelettiques par le procédé selon l'invention, pour la préparation d'une composition thérapeutique est caractérisée en ce que ladite composition est administrée par voie intra-veineuse ou par transplantation.

Les légendes des figures et exemples qui suivent sont destinés à illustrer l'invention.

### LEGENDES DES FIGURES

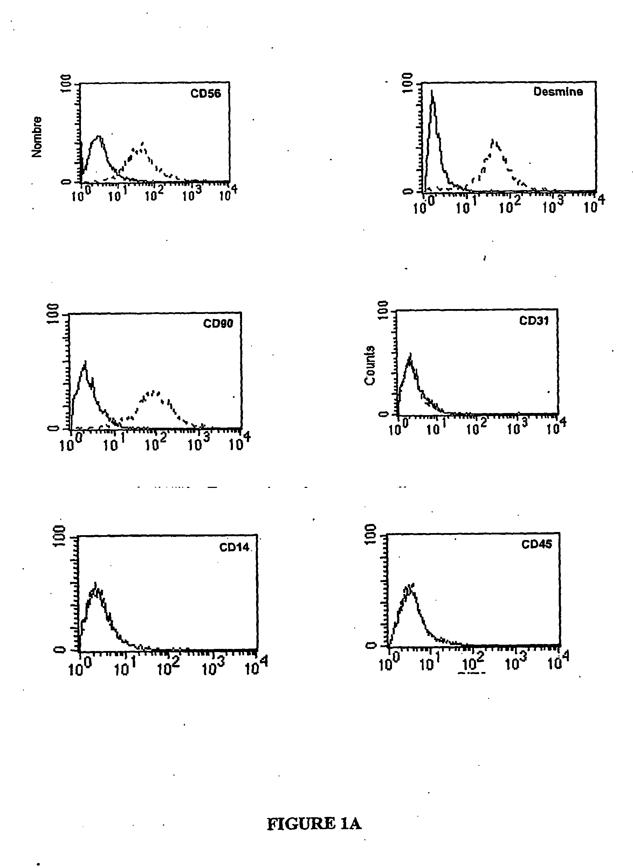

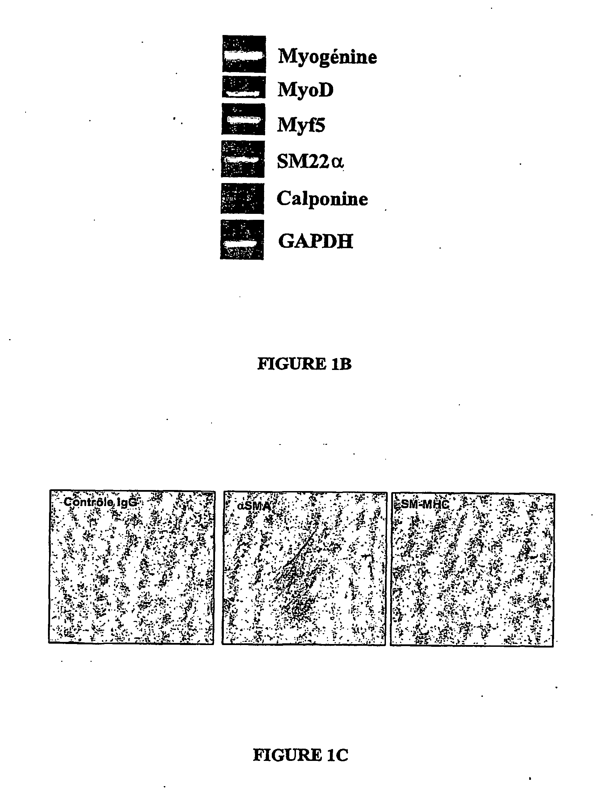

**Figures 1A à 1C****.** Caractérisation des cellules de muscle squelettique cultivées dans un milieu contenant du FGFb. (Figure 1A) L'analyse en cytométrie en flux démontre que ces cellules expriment CD56, la Desmine et CD90 mais qu'elles n'expriment pas CD31, CD14 et CD45. Dans chaque histogramme, la ligne noire correspond aux cellules marquées avec un anticorps de contrôle négatif La ligne en pointillés correspond aux cellules marquées avec l'anticorps spécifique du marqueur indiqué dans chaque histogramme. Ces histogrammes sont représentatifs de 6 échantillons. (Figure 1B) Analyse par RT-PCR (Figure 1C) Caractérisation des cellules de muscle squelettique cultivées par analyse immunocytochimique. Les cellules sont marquées soit avec un anticorps anti-IgG de contrôle, un anticorps anti-αSMA ou anti-SM-MHC suivi par un marquage avec un anticorps secondaire couplé à la péroxydase.

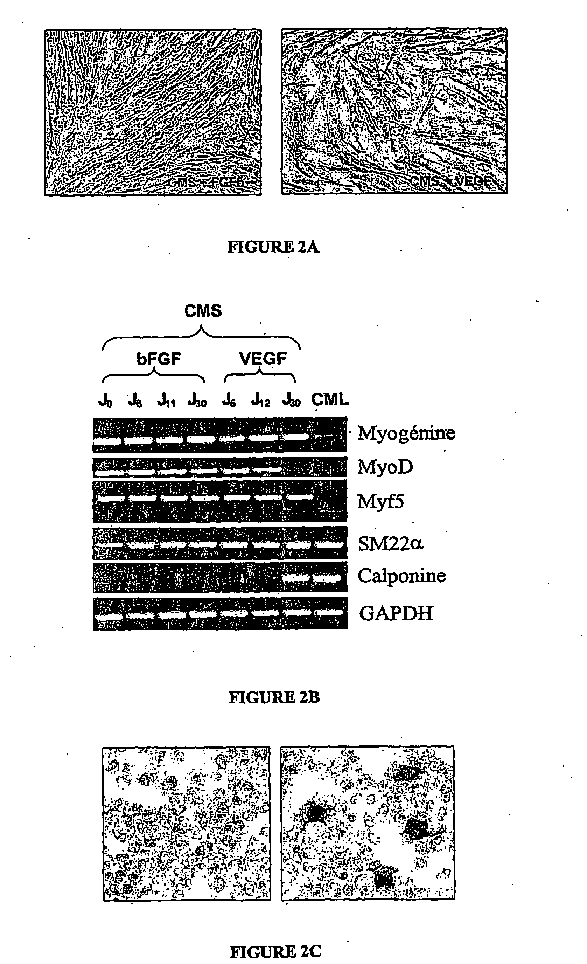

**Figures 2A à 2C.** (Figure 2A) Morphologie des cellules de muscle squelettique (CMS) dans du milieu contenant du FGFb ou du VEGF. (Figure 2B) Analyse par RT-PCR de l'expression de gènes spécifiques des cellules de muscle squelettique et lisse dans des CMS cultivées dans un milieu contenant du FGFb ou du VEGF. Les cultures ont été récoltées pour préparer l'ARN aux différents temps indiqués. La RT-PCR a été réalisée et les produits de PCR ont été analysés sur des gels d'agarose contenant du bromure d'éthidium. (Figure 2C) Mise en évidence de l'expression de SM-MHC par immunomarquage dans des CMS cultivées avec du VEGF pendant un mois. Les cellules ont été marquées soit avec un anticorps anti-IgG de contrôle, soit avec un anticorps anti-SM-MHC, suivi par un marquage avec un anticorps secondaire couplé à la péroxydase.

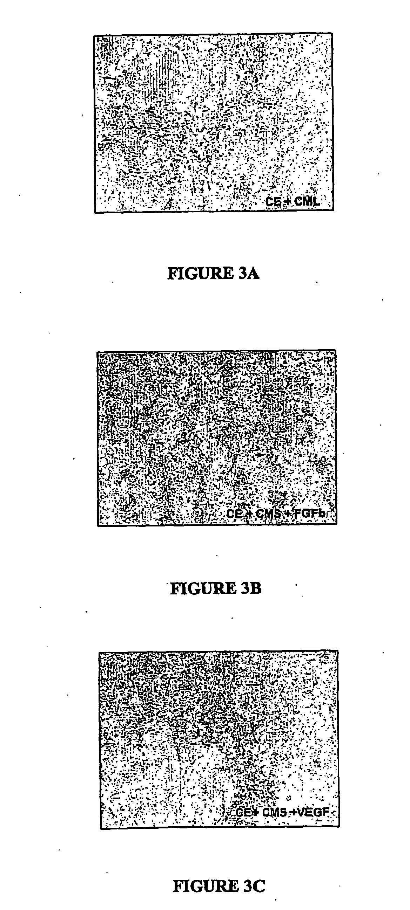

**Figures 3A à 3C****.** Photos prises au microscope à contraste de phase. Les cellules endothéliales (CE) et les cellules musculaires sont déposées ensemble à'la surface d'un gel de collagène. Au bout de 24-48 heures, les CE interagissent avec les CML, issues de la différenciation des précurseurs du sang de cordon ombilical (figure 3A), ou les CML obtenues après culture des cellules de muscle squelettique (figure 3C), pour former des réseaux. En revanche, les CMS ne peuvent pas former des réseaux dans ces conditions (figure 3B).

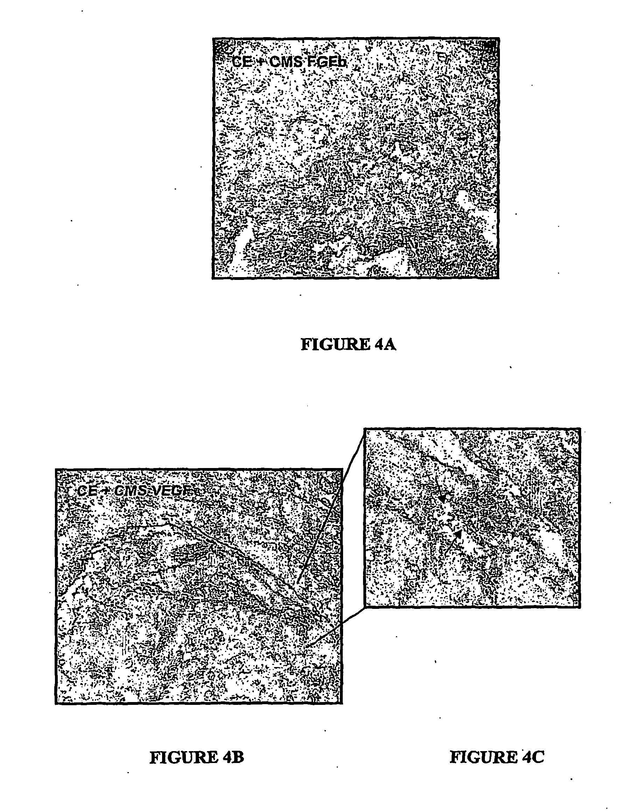

**Figures 4A à 4C****.** Coupes de matrigel, marquage HES.
   La co-injection des CE et des CML obtenues à partir des cellules du muscle squelettique, entraîne la formation, dans l'implant, de lacs vasculaires (figure 4B), avec la présence de globules rouges (ensemble de points au niveau des flèches, figure 4C à plus fort grossissement). En revanche, dans ces mêmes conditions, les CMS ne forment pas un réseau vasculaire fonctionnel (figure 4A).

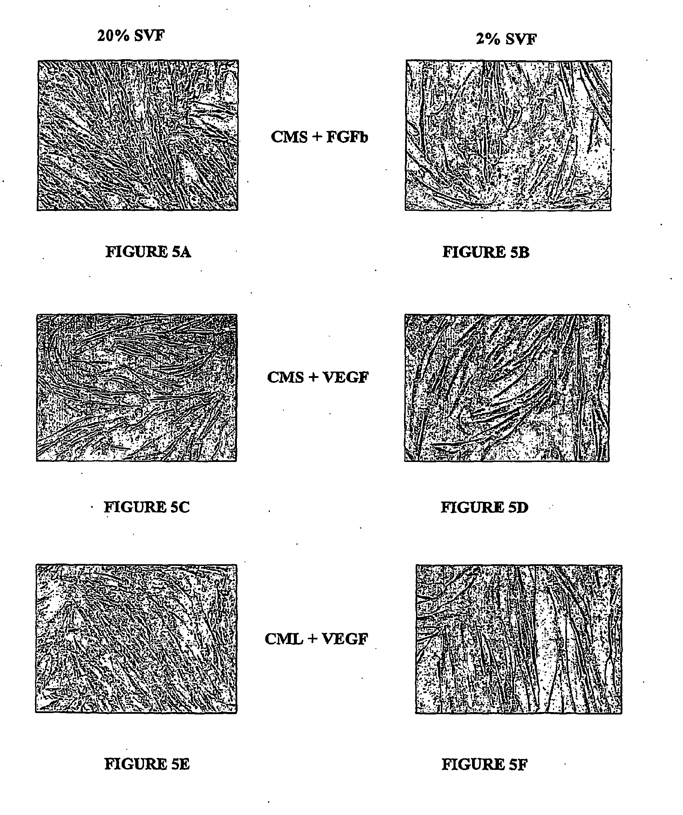

**Figures 5A à 5F****.** Photos prises au microscope à contraste de phase de CMS (Figures 5A à 5D) et de CML (Figures 5E et 5F) cultivées dans un milieu contenant 20 % de sérum de veau foetal (SVF) (Figures 5A, 5C et 5E) ou 2 % de SVF (Figures 5B, 5D et 5F). Pour induire la formation des cellules en myotubes, le milieu de culture des cellules arrivées à 80-90 % de confluence est changé par un milieu supplémenté à 2 % en SVF. L'abolissement de la formation de myotubes est associé à l'addition de VEGF dans le milieu (Figures 5C à 5F). Les CMS cultivées en présence de VEGF sont incapables de fusionner en myotubes multinucléés (Figure 5D).

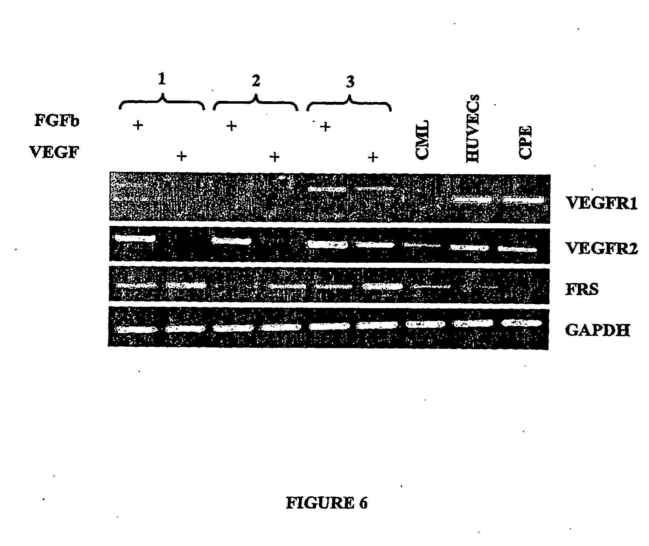

**Figure 6****.** Le degré de différenciation des CML est corrélé avec la diminution d'expression de VEGFR2 et l'augmentation de l'expression de FRS (Facteur de Réponse sérique). L'analyse par RT-PCR de 3 échantillons différents de CMS (1, 2 et. 3) cultivées en présence de FGFb ou de VEGF. Les cellules progénitrices endothéliales (CPE), obtenues comme décrit dans (16) sont utilisées à titre de contrôle positif pour l'expression des récepteurs du VEGF (VEGFR) et de contrôle négatif pour FRS. Quelles que soient les conditions de culture, les CMS et les CML n'expriment pas VEGFR1. Dans les CMS, le VEGF diminue l'expression de VEGFR2 mais stimule l'expression de l'ARNm du FRS.

### EXEMPLES

### METHODES

### Culture cellulaire

Les CML différenciées *ex vivo* à partir des précurseurs contenus dans le sang de cordon ont été obtenues comme décrit antérieurement (16). Elles ont été cultivées sur du collagène de queue de rat de type I (60 µg/ml, SIGMA), dans du milieu M199 (Gibco) supplémenté avec 20 % de sérum de veau foetal (SVF) à 20 %, 25 mM de tampon Hepes (Gibco) et d'une solution antibiotique et antifongique (Gibco) et d'hVEGF recombinant à 10 ng/ml (R & D Systems) à 37°C, et dans une atmosphère contenant 5 % de CO₂. Le milieu de culture est changé deux fois par semaine. Les CMS ont été cultivées comme décrit antérieurement (12). Pour induire la différenciation des cellules en myotubes, le milieu de culture des cellules à 80-90 % de confluence a été changé avec du milieu supplémenté à 2 % de SVF, 25 mM d'Hepes et une solution d'antibiotique et d'antifongique (Gibco).

### Immunocytochimie

Les cellules ont été mises en culture sur des plaques (« chamber slides », Lab-Techn, Poly Labo, Strasbourg, France) et fixées avec une solution d'acétone à froid à 90 %. Des anticorps primaires ont été utilisés. Un anticorps monoclonal murin antiaSMA humaine (1A4, DAKO) et un anticorps monoclonal murin anti-chaîne lourde de la myosine de muscle lisse humain (SMMS-1, DAKO). Le kit (DAKO) EnVision™ System Peroxydase (DAB) a été utilisé pour révéler l'αSMA et la SM-MHC. Les cellules ont été en final contrecolorées avec l'hématoxyline.

### Cytométrie de flux

Un aliquot de cellules a été directement marqué avec des anticorps dirigés contre CD31 (5.6E, Coulter), CD45/CD14 (2D1, MφP9, Becton Dickinson), CD56, et CD90. Les cellules ont été marquées avec un anticorps anti-Desmine (D33, DAKO) après une étape de perméabilisation avec le réactif de perméabilisation Intraprep™ (Coulter). Après marquage les cellules ont été fixées avec du paraformaldéhyde à 1 % et analysées par cytométrie de flux (FACStar flow cytometer, Becton Dickinson).

### RT-PCR (Transcription inverse-Réaction de polymérisation en chaîne)

L'ARN total a été extrait avec le réactif RNAXEL^{R} (EUROBIO, Les Ulis, France) selon les instructions du fournisseur. La synthèse de l'ADNc a été réalisée avec le kit « 1 st strand cDNA synthesis kit for RT-PCR (AMV) » (Boerhinger Mannheim). Par conséquent, le fragment d'ADNc d'intérêt a pu être amplifié par PCR Le mélange PCR contenait un tampon de réaction 1X, 1,5 mM MgCl₂, 0,2 mM de mélange de déoxynucléotides, 0,5 unité de Taq polymérase et 0,2 µM d'amorces sens et antisens. Les amorces suivantes ont été utilisées pour la RT-PCR : GAPDH sens (SEQ ID NO: 1) : 5' -CCA TGG AGA AGG CTG GGG- 3', antisens (SEQ ID NO: 2) : 5' -CAA AGT TGT CAT GGA TGA CC- 3', calponine sens (SEQ ID NO: 3) : 5' -AGA-AGT-ATG-ACC-ACC-AGC- 3', antisens (SEQ ID NO: 4) : 5' -TAG-AGC-CCA-ATG-ATG-TTC-CG- 3', SM22α sens (SEQ ID NO: 5) : 5' -GCA-GTC-CAA-AAT-TGA-GAA-GA- 3', antisens (SEQ ID NO: 6) : 5' -CTG-TTG-CTG-CCC-ATT-TGA-AG- 3', Myogénine sens (SEQ ID NO: 7) : 5' -AGC-GCC-CCC-TCG-TGT-ATG- 3', antisens (SEQ ID NO: 8) : 5' -TGT-CCC-CGG-CAA-CTT-CAG-C- 3', MyoD sens (SEQ ID NO: 9) : 5' -CGG-CGG-CGG-AAC-TGC-TAC-GAA- 3', antisens (SEQ ID NO: 10) : 5' -GGG-GCG-GGG-GCG-GAA-ACT-T- 3', Myf5 sens (SEQ ID NO: 11) : 5' -ACC-ATG-GAT-CGG-CGG-AAG-G- 3', antisens (SEQ ID NO: 12) : 5' -AAT-CGG-TGC-TGC-CAA-CTG-GAG- 3', VEGF-R1 sens (SEQ ID NO: 13) : 5' -CGA CCT TGG TTG TGG CTG ACT- 3', antisens (SEQ ID NO: 14): 5' -CCC TTC TGG TTG GTG GCT TTG- 3', VEGF-R2 sens (SEQ ID NO: 15) : 5' -AAC AAA GTC GGG AGA GGA- 3', antisens (SEQ ID NO: 16) : 5' -TGA CAA GAA GTA GCC AGA AGA- 3', SRF sens (SEQ ID NO: 17) : 5' AGT-GTG-TGG-GGG-AGA-TTC-TG- 3' et antisens (SEQ ID NO:18) : 5' -TCT-CCC-TAG-CAA-CAG-CCC-TA- 3'.

### EXEMPLE 1 : Conditions de culture permettant l'obtention en grande quantité, chez l'homme, de CML à partir des cellules progénitrices ou des cellules différenciées du muscle squelettique

### A) Population cellulaire de départ :

Le procédé de l'invention porte sur un procédé d'obtention d'une population de cellules dont un type cellulaire dominant est le type de cellules musculaires lisses. Ce procédé pourra être appliqué soit directement sur les cellules de biopsies musculaires, soit après une première phase de différenciation des cellules de la biopsie en CMS et d'amplification de ces cellules. Les conditions d'obtention des biopsies musculaires et des CMS à partir de ces biopsies ainsi que leur caractérisation phénotypique sont définies dans la demande internationale de brevet publiée sous le N° WO 01/94555 (J.P. Marolléau et coll.). De plus, les cellules de la biopsie n'expriment pas CD31 et CD14.

A partir de quelques grammes d'une biopsie musculaire, il est possible d'obtenir plusieurs centaines de millions de CMS. Ces cellules expriment le CD56, la desmine et les gènes de la myogenèse tels que Myf5 et la myogénine. En revanche elles n'expriment pas le CD34, le CD 14, et les marqueurs spécifiques des CML tels que : la calponine et la SM-MHC. Ces cellules sont enfin capables de fusionner et de donner naissance à des myotubes multinucléés.

### B) Différenciation cellules musculaires squelettiques vers cellules musculaires lisses :

Les cellules de la biopsie, ou après différenciation en CMS, sont mises en culture dans le milieu MCDB ou M199 en présence dé VEGF seul ou avec d'autres facteurs de croissance (PDGF-BB, IGF1, FGFb, HGF ou TNFα).

Solutions et milieux utilisés :
Milieu A :
   - Milieu MCDB 120 (Ham et al., 1988) modifié : substitution de la L-valine par de la D-valine, élimination du rouge de phénol et de la thymidine.
Milieu B :
   - Milieu A + 20 % de sérum de veau foetal irradié + antibiotique (gentamycine à 50 µg par ml, ou à 100 UI/ml pour la pénicilline et 100 µg/ml pour la streptomycine).
Milieu C :
   - Milieu B + FGFb (10 ng/ml) + dexaméthasone à 1 µM.
Solution D : Tampon phosphate salin (PBS)
   (voir la demande de brevet internationale publiée sous le No. WO 01/94555 pages 24 et 25).
Milieu E : M199
Milieu F : M199 + 20% de sérum de veau foetal décomplémenté + Hépes (25 mM) + antibiotique (pénicilline, streptomycine et, si nécessaire, un antimycotique (tel que de la fongizone à 25 µg/ml, ou comme indiqué ci-avant).
Milieu G : Milieu F (M199 + SVF + Hépes + antibiotique) + VEGF (10 ng/ml).
Milieu H : Milieu B (MCDB + SVF + antibiotique + dexaméthasone) + VEGF (10 ng/ml).

Les milieux contenant les différents facteurs de croissance décrits plus haut.

L'expression des gènes associés à la différenciation en CMS ou CML, au cours du temps de culture, a été analysée par polymérisation en chaîne après reverse transcription (RT-PCR), cytométrie en flux et immunocytochimie. Ainsi, après un mois de culture dans le milieu M199 ou MCDB 120 contenant du VEGF, ces cellules expriment les ARN messagers et les protéines spécifiques des CML. Elles expriment donc la calponine et la SM-MHC. En parallèle, elles expriment beaucoup moins fortement la desmine et le CD56 et plus du tout MyoD. En revanche, l'expression des facteurs de transcription Myf5 et Myogénine persiste.

Les inventeurs ont également mis en évidence que ces modifications phénotypiques entraînaient des propriétés fonctionnelles différentes.

### EXEMPLE 2 : Différenciation des cellules musculaires squelettiques en cellules musculaires lisses

Des cellules de biopsie de muscle ont premièrement été mises en culture pour expansion dans un milieu contenant du FGFb tel que décrit précédemment (12). Pour caractériser le phénotype de ces cellules, des analyses en cytométrie en flux (FACS), par réaction de polymérisation en chaîne avec transcription inverse (RT-PCR) et par immunocytochimie ont été réalisées. L'analyse par FACS a démontré que la plupart de ces cellules sont positives pour CD56 (80,30 + 19,50 %), la desmine (92,30 + 8,48 %) et CD90 (91,32 + 10,19 %) et négatives pour le marqueur endothélial CD31, le marqueur de monocytes CD14, et le marqueur de leucocytes CD45 (figure 1A). L'analyse par RT-PCR a démontré que les cellules expriment les marqueurs liés aux cellules myogéniques tels que Myf5, MyoD et Myogenin (figure 1B). Les cellules expriment également des marqueurs spécifiques des cellules du muscle lisse SM22α (figure 1B) et αSMA (figure 1C). Mais certaines isoformes de cellules de muscle lisse ont été détectées dans des cellules de muscle squelettique en développement ou en régénération (14, 15). Les cellules n'expriment pas de marqueurs de cellules de muscle lisse différenciées tels que la calponine (figure 1B) et SM-MHC (figure 1 C).

Les cellules ont ensuite été mises en culture dans un milieu contenant du VEGF (10 ng/ml). Après 7 jours, des changements de morphologie cellulaire ont été observés (figure 2A). La technique de RT-PCR a été utilisée pour comparer les changements d'expression de gènes pendant la culture entre les cellules de muscle squelettique et de muscle lisse. Cette analyse a été réalisée sur de l'ARN obtenu à partir de cellules aux jours 0, 6, 11 ou 12 et 30 après mise en culture, et les résultats sont présentés sur la figure 2B. Il a été observé que les gènes codant pour SM22α, Myogenin et Myf 5 sont exprimés à un niveau similaire, quelles que soient les conditions de culture et pendant toute la durée de la culture. Les cellules de muscle squelettique (CMS) mises en culture avec du FGFb ne montrent aucune expression de calponine. Mais après un mois de culture avec du VEGF, ces cellules expriment de l'ARNm de calponine et simultanément n'expriment plus MyoD (figure 2B). L'expression de SM-MHC confirme que ces cellules ont adopté un phénotype de cellules de muscle lisse (figure 2C). Du fait de la variabilité dans l'expression de gènes structuraux dans les cellules de muscle lisse (CML), il est généralement soutenu que pour qu'une cellule soit caractérisée comme une CML différenciée, il est nécessaire de démontrer l'expression de plusieurs isoformes de gènes structuraux associés au muscle lisse. Ainsi, l'expression de SM22α, de la calponine et de SM-MHC dans les CMS est une forte indication que certaines des cellules de la culture ont adopté une identité de CML différenciée. De façon à définir les meilleures conditions de culture, d'autres facteurs de croissance connus pour induire la différenciation ou la prolifération des CML ont été testés. Des conditions de culture dans lesquelles du PDGF BB et/ou du FGFb et/ou du HGF et/ou du TGFβ et/ou du IGFI a été ajouté au VEGF ont donc été testées. Mais aucun effet significatif sur la différenciation de CMS en CML ou sur la prolifération n'a été observé.

Frid M.G. et aL (13) ont démontré que l'endothélium bovin mature contient des cellules qui, *in vitro,* peuvent acquérir un phénotype de CML par un processus de transdifférenciation. Il a été confirmé ici par analyse par FACS que les cellules en culture ne sont pas contaminées par des cellules endothéliales (CE). Elles n'expriment pas de marqueurs liés aux cellules endothéliales tels que CD31 (figure 1A). En outre, on peut faire l'hypothèse que le phénomène observé ne représente pas une simple contamination par des CML provenant d'une source externe. En effet, toutes les biopsies testées, qui montrent l'expression des gènes associés au muscle squelettique Myogenine, MyoD, Myf5 et Desmine, subissent une différenciation en muscle lisse.

### EXEMPLE 3 : Essais fonctionnels

L'acquisition de marqueurs de cellules de muscle lisse ne signifie par nécessairement que ces cellules sont capables de se différencier en CML matures.

### A) Culture en gel de collagène de type I (culture en 3D)

### Protocole :

La culture en gel de collagène de type I (culture en 3D) (BD Biosciences, Bedfoed, MA) a été réalisée selon les recommendations du fournisseur, à savoir :
   0,5 ml de collagène de queue de rat de type I à 1 mg/ml (Becton Dickinson) est coulé dans des boîtes de culture de 35 mm de diamètre (Nunc, Fisher Scientific, Elancourt, France) et laissé à polymériser pendant 1 heure à 37°C. 400 000 cellules au total (200 000 de chacun des types cellulaires quand mélange des cellules endothéliales avec les cellules musculaires) sont alors déposées à la surface du gel et mises en culture pendant 24 heures dans les différentes conditions de culture. La formation de réseaux vasculaires est ensuite observée au microscope à contraste de phase et avec une caméra « charge-coupled » Kappa CF11DSP.

### Résultats (voir figures 3A à 3C) :

La capacité de ces cellules à s'organiser dans une culture tridimensionnelle de collagène a été analysée. Il a été démontré que les cellules endothéliales (CE) et les CML interagissent entre elles pour former des réseaux de type capillaire *in vitro* en culture tridimensionnelle. La capacité de CMS cultivées avec du FGFb, et de CMS cultivées avec du VEGF à s'associer avec des CE et à former des réseaux de type capillaire in *vitro* a été comparée. On observe que les CMS cultivées avec du VEGF sont capables d'interagir avec les CE formant des réseaux tubulaires compacts (figure 3C), alors que les CMS cultivées avec du FGFb n'interagissent pas avec les CE dans les mêmes conditions et ne forment aucun réseau de vaisseaux (voir figure 3B).

### B) Modèle matrigel

La capacité de chaque type cellulaire, CMS avec FGFb ou avec VEGF, à former des structures de vaisseaux tubulaires dans un modèle d'implant matrigel dans des souris NOD-SCID immunodéprimées a été testée.

### Protocole :

J0 : Un implant de 0,2 ml de matrigel (BD Biosciences) (contenant 0,5 mg/ml de FGFb) est injecté en sous-cutané sur le dos des souris immunodéprimées NOD-SCID.
J1 : Le matin, les souris sont irradiées de façon sub-létale (325 rad). L'après midi, 500. 000 cellules sont injectées en intraveineux via la veine caudale.
J10 : Les animaux sont sacrifiés, l'implant est récupéré et inclus en paraffiné. Une coloration HES (hémaline, éosine, safran) a été réalisée puis examinée (grandissement 4x, 40x).

### Résultats (voir figures 4A à 4C) :

Ces résultats ont été obtenus et reproduits à partir de CMS provenant de 3 patients différents ou à partir de biopsies issues de 5 patients différents.

Dans l'implant matrigel, l'administration de CE et de CML ou de CE et de CMS cultivées avec du VEGF conduit à la formation de nombreuses structures de type tubulaire et la présence d'érythrocytes est mise en évidence dans la lumière, démontrant l'existence d'une structure vasculaire fonctionnelle (figures 4B et 4C). Inversement, l'administration de CE et de CMS cultivées avec du FGFb ne conduit à la formation d'aucune structure de type tubulaire et induit la formation d'agrégats de cellules désorganisés (figure 4A).

### C) Les CMS cultivées en présence de VEGF ne fusionnent plus en myotubes multinucléés

### (Voir figures 5A à 5F)

La fusion des myoblastes individuels en myotubes multinucléés constitue la différenciation terminale des CMS. La formation de myotubes a été examinée en mettant en culture des CMS, avec du FGFb ou du VEGF, à la même densité initiale, et en changeant ensuite les conditions de culture pour un milieu avec 2 % de sérum de veau foetal. Dans ces conditions, des myotubes apparaissent 10 jours après la mise en culture. Contrairement aux CMS cultivées avec du FGFb (figure 5B), les CMS cultivées avec du VEGF (figure 5D), de même que les CML (figure SF), sont incapables de fusionner en myotubes multinucléés. Ces cellules ont donc perdu la capacité à former des myotubes multinucléés.

### D) Le VEGF est impliqué dans l'induction de la transition du phénotype CMS à CML en augrnentant l'expression du facteur de réponse sérique FRS (dénommé aussi SRF pour« serum response factor » )

Le VEGF est un régulateur majeur de la formation des vaisseaux sanguins pendant le développement de l'organisme et chez l'adulte. Afin d'explorer la voie de transduction du signal possible pour le VEGF, l'expression des récepteurs VEGFR1 et VEGFR2 a été analysée dans les CMS et les CML. L'analyse par RT-PCR met en évidence que les CMS expriment une grande quantité de VEGFR2. Mais lorsque ces cellules sont cultivées dans un milieu contenant du VEGF une diminution de l'expression de VEGFR2 est observée (figure 6). Et, quelles que soient les conditions de culture, nous n'avons pas mis en évidence la détection d'expression de VEGFR1. Ces résultats suggèrent ainsi le rôle de VEGFR2 dans la médiation de la transdifférenciation des CMS en CML stimulée par le VEGF. Le FRS est un régulateur clé de nombreux gènes de réponse précoce cellulaire qui sont connus pour être impliqués dans la croissance et la différenciation cellulaire. Certains résultats suggèrent qu'un ou plusieurs cofacteurs de FRS restreints à la lignée CMS ou CML pourraient fonctionner en concert avec le FRS pour activer la transcription de gènes restreints à des lignées. Dans le but de comprendre les mécanismes intervenant dans la différenciation des CMS en CML, l'expression de FRS a été comparée dans les cellules avant et après addition de VEGF. Il a été observé que lorsque les CMS sont cultivés dans un milieu contenant du VEGF l'expression de l'ARNm FRS était augmentée (figure 6).

### Références

1: Gussoni E. et al. : Dystrophin expression in the mdx mouse restored by stem cell transplantation. Nature 1999, 401:390-394.
2: Jackson KA. Et al. From the cover : hematopoietic potential of stem cells isolated from murine skeletal muscle. PNAS 1999, 96:14482-14486.
3: Tamaki T. et al. : Identification of myogenic-endothelial progenitor cells in the interstitial spaces of skeletal muscle. J Cell Biol 2002 ; 157:571-577.
4: Hwang JH. et aL : Differentiation of stem cells isolated from rat smooth muscle. Mol Cells 2004 ; 17(1):57-61.
5: Benjamin L.E. et aL : Selective ablation of immature blood vessels in established human tumors follows vascular endothelial growth factor withdrawal. J Clin Invest 1999 ; 103:159-165.
6: Morikawa S. et aL : Abnormalities in pericytes on blood vessels and endothelial sprouts in tumors. Am J Pathol 2002 ; 160:985-1000.
7: Berger M. et aL : Regulator of G protein signaling-5 induction in pericytes coincides with active vessel remodeling during neovascualrization. Blood 2004 epub le 30/09/04.
8: Bergers G. et aL : benefits of targeting both pericytes and endothelial cells in the tumor vacsulature with kinase inhibitors. J Clin Invest-2003 ; 111:1287-1295.
9: Jain R.K. : Normalizing of tumor vasculature: an emerging concept in antiangiogenic therapy. Science 2005 ; 307:58-62.
10: Ganss R. et al. : Combination of T-Cell therapy and trigger of inflammation induces remodeling of the vasculature and tumor eradication. Cancer Res 2002 ; 62:1462-1470.
11: Rajantie I. et aL : Adult bone marrow-derived cells recruited during angiogenesis comprise precursors for periendothelial vascular mural cells. Blood 2004 ; 104:2084-2086.
12: Menasché P, Hagège AA, Vilquin JT, et al. Autologous skeletal myoblast transplantation for severe postinfarction left ventricular dysfunction. J. Am. Coll. Cardiol. 41, 1078-1083 (2003).
13: Frid MG, Kale VA, Stenmark KR. Mature vascular endothelium can give rise to smooth muscle cells via endothelium-mesenchymal transdifferentiation. In vitro analysis. Circ. Res. 90, 1189-1196 (2002).
14: Li L, Miano JM, Cserjesi P, Oison EN. SM22 alpha, a marker of adult smooth muscle, is expressed in multiple myogenic lineages during embryogenesis. Circ. Reis. 78, 188-195 (1996).
15: Woodcock-Mitchell J, Mitchell JJ, Low RB, Kieny M, Sengel P, Rubbia L, Skalli O, Jackson B, Gabbiani B. α-Smooth mucle actin is transiently expressed in embryonic rat cardiac and skeletal muscles. Differentiation 39, 151-166 (1998).
16: Le Ricousse-Roussanne S, Barateau V, Contreres JO, et al. Ex vivo differentiated endothelial and smooth muscle cells from human cord blood progenitors home to the angiogenic tumor vasculature. Cardiovas. Res. 62, 176-184 (2004).

## Revendications

1. Procédé d'obtention *in vitro* d'une population de cellules comprenant essentiellement des cellules musculaires lisses humaines (hCML) exprimant la calponine et la chaîne lourde de la myosine musculaire lisse (SM-MHC) à partir d'un échantillon de cellules de biopsie musculaire squelettique humaine ou à partir de cellules de biopsies musculaires humaines différenciées *in vitro* en cellules musculaires squelettiques (hCMS),
- lesdites cellules de biopsies musculaires humaines n'exprimant pas le CD31 et le CD 14, et, le cas échéant, les marqueurs des lymphocytes B et T, et
- lesdites hCMS exprimant le CD56, la desmine et un gène de la myogénèse choisi dans le groupe de gènes constitué du gène MyoD, Myf5 et la myogénine, et sont capables de générer des myotubes multinucléés,
**caractérisé en ce qu'**il comprend les étapes suivantes :
A) la mise en culture desdites cellules de biopsies musculaires humaines myoblastiques dans un milieu de culture comprenant du VEGF, ladite culture étant réalisée en absence de CML issues de vessie ; et
B) la récupération des hCML obtenues à l'étape A).

2. Procédé selon la revendication 1, **caractérisé en ce que** lesdites hCMS exprimant le CD56 et la desmine, expriment les gènes MyoD, Myf5 et la myogénine.

3. Procédé selon la revendication 1 ou 2, **caractérisé en ce que** lesdites hCMS n'expriment pas le CD34 et le CD14.

4. Procédé selon l'une des revendications 1 à 3, **caractérisé en ce que** lesdites hCMS n'expriment pas la calponine et la SM-MHC.

5. Procédé selon l'une des revendications 1 à 4, **caractérisé en ce que** lesdites hCML obtenues à l'étape A) expriment la calponine et la SM-MHC.

6. Procédé selon l'une des revendications 1 à 5, **caractérisé en ce que** lesdites hCML obtenues à l'étape A) n'expriment pas le gène MyoD.

7. Procédé selon l'une des revendications 1 à 6, **caractérisé en ce que** lesdites hCML obtenues à l'étape A) expriment Myf5 et la myogénine.

8. Procédé selon l'une des revendications 1 à 7, **caractérisé en ce que** ledit milieu de culture utilisé à l'étape A) comprend en outre au moins un facteur de croissance choisi dans le groupe de facteurs de croissance constitué du PDGF-BB, IGF1, FGFb, HGF, TNFα et TGFβ.

9. Procédé selon l'une des revendications 1 à 8 dans lequel lesdites hCML sont obtenues à partir d'un échantillon de cellules de biopsies musculaires humaines différenciées *in vitro* en cellules musculaires squelettiques (hCMS), **caractérisé en ce que** lesdites hCMS sont obtenues à partir d'un échantillon de cellules de biopsie musculaire humaine par un procédé comprenant les étapes suivantes :
a) éminçage de ladite biopsie musculaire,
b) dissociation enzymatique des fibres et cellules musculaires et séparation des cellules individualisées par filtradon,
c) mise en culture des cellules d'origine musculaire ainsi obtenues dans un réacteur de culture de cellules adhérentes en présence d'un milieu de croissance et/ou de différenciation suivie le cas échéant d'une ou plusieurs phases d'expansion,
d) identification des types cellulaires présents aux différents stades de la culture par l'analyse de marqueurs cellulaires spécifiques,
e) choix du stade de culture pendant lequel le type cellulaire recherché est en proportion dominante dans la population de cellules,
f) récolte d'une population de cellules au stade de culture choisi en e),
g) le cas échéant, congélation des cellules récoltées à l'étape f).

10. Procédé selon la revendication 9, **caractérisé en ce que** :
- à l'étape b), on effectue :
- un lavage des éminçats dans un milieu A suivi de la dissociation enzymatique en présence de libérase dudit éminçat ;
- la séparation des cellules individualisées ainsi obtenues par filtration sur tamis suivie d'une centrifugation ; et
- le lavage dans un milieu B du culot cellulaire ainsi obtenu ;
- à l'étape c), on effectue :
- la mise en culture des cellules obtenues à l'étape b) sur un plateau de culture dans un milieu C jusqu'à obtenir un degré de confluence d'environ 20 à 50 % ou jusqu'à l'apparition des premiers myotubes, puis le lavage des cellules en tampon PBS, en SVF puis en milieu C, la culture en milieu C) sur des unités de plateaux élargis ou multi-étagés pouvant être de nouveau réalisée pour atteindre un degré de confluence d'environ 90 % ou l'apparition des premiers myotubes,
- l'élimination du milieu de culture C et son remplacement par un milieu D la veille de la récolte desdites cellules,
- le lavage des cellules ainsi obtenues en PBS puis en milieu A ; et,
- le cas échéant, la concentration desdites cellules ainsi obtenues en milieu A supplémentée de sérum albumine humaine à 0,5 % (P/V) à la fin de l'étape f) ;
- à l'étape g), la congélation desdites cellules ainsi obtenues à l'étape f) est réalisée en milieu A supplémentée de sérum albumine humaine à 4 % (P/V) et en DMSO à 7,5 % (V/V), leur décongélation à 37°C, et après un lavage en milieu A leur suspension dans le milieu de culture,
et dans lesquelles étapes lesdits milieux A. B. C et D sont les suivants :
Milieu A :
- Milieu MCDB 120 (Ham et al., 1988) modifié : substitution de la L-valine par
de la D-valine, élimination du rouge de phénol et de la thymidine,
Milieu B :
- Milieu A + 20 % de sérum de veau foetal irradié + antibiotique,
Milieu C :
- Milieu B + FGFb (10 ng/ml) + dexaméthasone à 1 µM,
Solution ou milieu D : Tampon phosphate salin (PBS).

11. Procédé selon la revendication 9, **caractérisé en ce que** le stade de culture pendant lequel le type cellulaire hCMS recherché est en proportion significative dans la population de cellules est déterminé par l'apparition d'une population cellulaire de phénotypes CD56+ représentant au moins 50 % de la population générale.

12. Procédé selon la revendication 11 dans lequel ladite population cellulaire de phénotypes CD56+ représentant au moins 50 % de la population générale possède en outre l'un au moins des phénotypes, de préférence au moins 2, 3 et les 4 phénotypes, choisi dans le groupe de phénotypes constitué de CD 10+, CD 13+, desmine+ et HLA de classe 1.

## Claims

1. Method for obtaining *in vitro* a population of cells comprising essentially human smooth muscle cells (hSMC) expressing calponin and smooth muscle myosin heavy chain (SMMHC) from a sample of human skeletal muscle biopsy cells or from human muscle biopsy cells differentiated *in vitro* into skeletal muscle cells (hSkMC) .
- said human muscle biopsy cells not expressing CD31 and CD14 and, if applicable, lymphocyte markers B and T, and
- said hSkMC expressing CD56, desmin and a myogenesis gene selected from the group of genes constituted by the genes MyoD, Myf5 and myogenin, and able to generate multinuclear myotubes,
**characterised in that** it comprises the following steps:
A) growing said myoblastic human muscle biopsy cells in a culture medium comprising VEGF, said culture being carried out in the absence of bladder SMC; and
B) recovery of the hSMC obtained in step A).

2. Method according to claim 1, **characterised in that** said hSkMC expressing CD56 and desmin express the genes MyoD, Myf5 and myogenin.

3. Method according to claims 1 or 2, **characterised in that** said hSkMC do not express CD34 and CD14.

4. Method according to one of claims 1 to 3, **characterised in that** said hSkMC do not express calponin and SMMHC.

5. Method according to one of claims 1 to 4, **characterised in that** said hSMC obtained at step A) express calponin and SMMHC.

6. Method according to one of claims 1 to 5, **characterised in that** said hSMC obtained at step A) do not express the gene MyoD.

7. Method according to one of claims 1 to 6 **characterised in that** said hSMC obtained at step A) express Myf5 and myogenin.

8. Method according to one of claims 1 to 7, **characterised in that** said culture medium used at step A) further comprises at least one growth factor selected from the group of growth factors consisting of PDGF-BB, IGF1, FGFb, HGF, TNFα and TGF13.

9. Method according to any of claims 1 to 8, wherein said hSMC are obtained from a sample of human muscle biopsy cells differentiated *in vitro* into skeletal muscular cells (hSkMC), **characterised in that** said hSkMC are obtained from a sample of human muscle biopsy cells by a method comprising the following steps:
a) mincing of said muscle biopsy,
b) enzymatic dissociation of the fibres and muscle cells and separation of the individualised cells by filtration,
c) growing the cells of muscle origin thus obtained in an adherent cells culture reactor in the presence of a growth medium and/or differentiation medium followed if appropriate by one or several expansion phases,
d) identification of the cell types present at the different stages of the culture by analysis of specific cell markers,
e) choosing the stage of culture during which the required cell type is a dominant proportion of the cell population,
f) harvesting a population of cells at the culture stage selected in e),
g) if appropriate, deep freezing the cells harvested in step f).

10. Method according to claim 9, **characterised in that**:
- at step b), the following steps are carried out:
- - washing the mincings in a medium A followed by enzymatic dissociation of said mincings in the presence of liberase;
- - separating the individualised cells thus obtained by filtering through a sieve followed by centrifugation; and
- - washing the cell peller thus obtained in a medium B;
- at step c), the following steps are carried out:
- - growing the cells obtained at step b) on a culture plate in a medium C until they are approximately 20 to 50% confluent or until the first myotubes appear, then washing the cells in PBS, FCS then in medium C, and then they can be cultured again in medium C) on enlarged or multi-stage plates or in culture flasks to achieve a degree of confluence of about 90% or the appearance of the first myotubes,
- - removing the culture medium C and replacing it by medium D the day before harvesting said cells,
- - washing the cells thus obtained in PBS then in medium A, and
- - if necessary, concentrating said cells thus obtained in medium A supplemented with 0.5% (P/V) human albumin serum at the end of step f);
- at step g) deep freezing said cells thus obtained at step f) is carried out in medium A supplemented with 4% (P/V) human albumin serum and with 7.5% (V/V) DMSO, thawing them at 37°C and then, after washing in medium A, suspending them in the culture medium,
and in which steps said media A, B, C, and D are the following:
Medium A:
- Modified MCDB 120 medium (Ham *et al.,* 1988): L-valine substituted by D-valine, removal of phenol red and thymidine,
Medium B:
- Medium A + 20% irradiated foetal calf serum + antibiotic,
Medium C:
- Medium B + FGFb (10 ng/ml) + 1 µM dexamethasone, Solution or medium D: Phosphate buffered saline (PBS).

11. Method according to claim 9, **characterised in that** the culture stage during which the required hSkMC cell type is a significant proportion of the cell population is determined by the appearance of a CD56+ phenotype cell population representing at least 50% of the general population.

12. Method according to claim 11, wherein said CD56+ phenotype cell population representing at least 50% of the general population further possesses at least one of the phenotypes, preferably at least 2, 3 and the 4 phenotypes, selected in the group of phenotypes composed of CD10+, CD13+, desmin+ and class 1 HLA.

## Patentansprüche

1. Verfahren zur Gewinnung einer Population von Zellen *in vitro,* umfassend im Wesentlichen humane Glattmuskelzellen (hCML), welche Calponin und die schwere Kette des glattmuskulären Myosins (SM-MHC) exprimieren, ausgehend von einer Probe von biopsierten humanen Skelettmuskelzellen oder ausgehend von biopsierten humanen Muskelzellen, welche *in vitro* zu Skelettmuskelzellen (hCMS) differenziert sind,
- wobei die biopsierten humanen Muskelzellen kein CD31 und CD14 und gegebenenfalls keine B- und T-Lymphozyten-Marker exprimieren, und
- wobei die hCMS CD56, Desmin und ein Gen der Myogenese exprimieren, welches ausgewählt aus der Gruppe von Genen bestehend aus dem Gen MyoD, Myf5 und Myogenin, und wobei die hCMS in der Lage sind, mehrkernige Myotuben zu erzeugen,
**dadurch gekennzeichnet, dass** das Verfahren die folgenden Schritte umfasst:
A) Kultivieren der biopsierten humanen myoblastischen Muskelzellen in einem VEGF enthaltenden Kulturmedium, wobei die Kultivierung in Abwesenheit von CML, die von der Harnblase stammen, durchgeführt wird; und
B) Ernten der in Schritt A) erhaltenen hCML.

2. Verfahren nach Anspruch 1, **dadurch gekennzeichnet, dass** die CD56 und Desmin exprimierenden hCMS die Gene MyoD, Myf5 und Myogenin exprimieren.

3. Verfahren nach Anspruch 1 oder 2, **dadurch gekennzeichnet, dass** die hCMS kein CD34 und CD14 exprimieren.

4. Verfahren nach einem der Ansprüche 1 bis 3, **dadurch gekennzeichnet, dass** die hCMS kein Calponin und SM-MHC exprimieren.

5. Verfahren nach einem der Ansprüche 1 bis 4, **dadurch gekennzeichnet, dass** die in Schritt A) erhaltenen hCML Calponin und SM-MHC exprimieren.

6. Verfahren nach einem der Ansprüche 1 bis 5, **dadurch gekennzeichnet, dass** die in Schritt A) erhaltenen hCML das Gen MyoD nicht exprimieren.

7. Verfahren nach einem der Ansprüche 1 bis 6, **dadurch gekennzeichnet, dass** die in Schritt A) erhaltenen hCML Myf5 und Myogenin exprimieren.

8. Verfahren nach einem der Ansprüche 1 bis 7, **dadurch gekennzeichnet, dass** das in Schritt A) verwendete Kulturmedium ferner mindestens einen Wachstumsfaktor enthält, der ausgewählt ist aus der Gruppe von Wachstumsfaktoren bestehend aus PDGF-BB, IGF1, FGFb, HGF, TNFα und TGFβ.

9. Verfahren nach einem der Ansprüche 1 bis 8, wobei die hCML ausgehend von einer Probe von biopsierten humanen Muskelzellen, welche *in vitro* in Skelettmuskelzellen (hCMS) differenziert sind, erhalten werden, **dadurch gekennezeichnet, dass** die hCMS ausgehend von einer Probe von biopsierten humanen Muskelzellen mittels eines Verfahrens erhalten werden, welches die folgenden Schritte umfasst:
a) Aufschneiden der Muskelbiopsie,
b) enzymatisches Zersetzen der Muskelzellen und -fasern und Abtrennen der vereinzelten Zellen durch Filtration,
c) Kultivieren der so erhaltenen Zellen muskulärer Herkunft in einem Kulturreaktor mit adhärierenden Zellen in Gegenwart eines Wachstums- und/oder Differenziationsmediums, gegebenenfalls gefolgt von einer oder mehreren Expansionsphasen,
d) Identifizieren der in verschiedenen Stadien der Kultivierung vorliegenden Zelltypen durch Analyse spezifischer Zellmarker,
e) Auswählen desjenigen Stadiums der Kultivierung, während dem der gesuchte Zelltyp den überwiegenden Anteil der Zellpopulation darstellt,
f) Ernten einer Zellpopulation bei dem in e) ausgewählten Stadium der Kultivierung,
g) gegebenenfalls Einfrieren der in Schritt f) geernteten Zellen.

10. Verfahren nach Anspruch 9, **dadurch gekennzeichnet, dass**
- in Schritt b) durchgeführt wird:
- Waschen der Schnitte in einem Medium A gefolgt vom enzymatischen Zersetzen der Schnitte in Gegenwart von Liberase;
- Abtrennen der so erhaltenen vereinzelten Zellen mittels Siebfiltration gefolgt von einer Zentrifugation; und
- Waschen des so erhaltenen Zellpellets in einem Medium B;
- in Schritt c) durchgeführt wird:
- Kultivieren der in Schritt b) erhaltenen Zellen auf einem Kulturplateau in einem Medium C bis ein Konfluenzgrad von etwa 20 bis 50% erhalten wird oder bis zum Erscheinen der ersten Myotuben, dann Waschen der Zellen in PBS-Puffer, in SVF und dann in dem Medium C, wobei die Kultur in dem Medium C auf erweiterten oder mehrstufigen Plateaueinheiten erneut durchgeführt werden kann, um einen Konfluenzgrad von etwa 90% oder das Erscheinen der ersten Mytuben zu erreichen,
- Entfernen des Kulturmediums C und Ersetzen durch ein Medium D am Vortag der Ernte der Zellen,
- Waschen der so erhaltenen Zellen in PBS und dann in dem Medium A; und
- gegebenenfalls Konzentrieren der so erhaltenen Zellen in dem Medium A, welches mit 0,5% (m/V) humanem Serumalbumin supplementiert ist, am Ende von Schritt f);
- in Schritt g) das Einfrieren der so in Schritt f) erhaltenen Zellen in dem Medium A durchgeführt wird, welches mit 4% (m/V) humanem Serumalbumin und mit 7,5% (V/V) DMSO supplementiert ist, deren Auftauen bei 37°C, und nach einem Waschen in dem Medium A deren Suspension in dem Kulturmedium,
und wobei in den Schritten die Medien A, B, C und D die Folgenden sind: Medium A:
- modifiziertes Medium MCDB 120 (Ham et al., 1988):
Ersetzen von L-Valin durch D-Valin, Weglassen von
Phenolrot und Thymidin,
Medium B:
- Medium A + 20% bestrahltes fetales Kälberserum +
Antiobiotikum,
Medium C:
- Medium B + FGFb (10 ng/ml) + 1 µM Dexamethason, Lösung oder Medium D: phosphatgepufferte Salzlösung (PBS).

11. Verfahren nach Anspruch 9, **dadurch gekennzeichnet, dass** das Stadium der Kultivierung, während dem der gesuchte Zelltyp hCMS in signifikantem Anteil in der Zellpopulation vorliegt, durch das Erscheinen einer Zellpopulation vom Phänotyp CD56+, die mindestens 50% der allgemeinen Population darstellt, bestimmt wird.

12. Verfahren nach Anspruch 11, wobei die Zellpopulation vom Phänotyp CD56+, die mindestens 50% der allgemeinen Population darstellt, ferner mindestens einen der Phänotypen, bevorzugt mindestens 2, 3 und alle 4 Phänotypen aufweist, die ausgewählt sind aus der Gruppe von Phänotypen bestehend aus CD10+, CD13+, Desmin+ und HLA der Klasse 1.
